# EUROPEAN PATENT SPECIFICATION

(11) **EP 4 701 613 B1**
(45) Date of publication and mention of the grant of the patent: **28.10.2026**
(21) Application number: 24718475.7
(22) Date of filing: 11.04.2024
(51) Int. Cl.: A61K 8/42, A61Q 19/00

(54) **COSMETIC USES FOR MAINTAINING AND RESTORING SKIN BARRIER FUNCTION**
KOSMETISCHE VERWENDUNGEN ZUR AUFRECHTERHALTUNG UND WIEDERHERSTELLUNG DER HAUTBARRIEREFUNKTION
UTILISATIONS COSMÉTIQUES POUR MAINTENIR ET RESTAURER LA FONCTION BARRIÈRE CUTANÉE

(30) Priority: 24.04.2023 EP 23169457
(43) Date of publication of application: 04.03.2026
(73) Proprietor: Unilever IP Holdings B.V., 6708 WH Wageningen (NL); Unilever Global IP Limited, Wirral, Merseyside CH62 4ZD (GB)
(72) Inventor: EVANS, Richard Livesey, 6708 WH Wageningen (NL); MAYES, Andrew Easson, 6708 WH Wageningen (NL); SMITH, Louise Victoria, 6708 WH Wageningen (NL); NANDOU, Lu, 6708 WH Wageningen (NL)
(74) Representative: Unilever Patent Group
(86) International application number: PCT/EP2024/059867
(87) International publication number: WO 2024/223320

(56) References cited:
- WO-A1-2009/016088
- US-A1- 2021 145 723
- ARÉVALO BÁRBARA ET AL: "Selective TASK-1 Inhibitor with a Defined Structure-Activity Relationship Reduces Cancer Cell Proliferation and Viability", vol. 65, no. 22, 15 November 2022 (2022-11-15), US, pages 15014 - 15027, XP093090159, ISSN: 0022-2623, Retrieved from the Internet <URL:https://pubs.acs.org/doi/pdf/10.1021/acs.jmedchem.1c00378> DOI: 10.1021/acs.jmedchem.1c00378
- KIM SANGHEE ET AL: "Solution-Phase Synthesis of a Library of Biaryl Amides Using Girard's Reagent T as an Acid Chloride Scavenger", JOURNAL OF COMBINATORIAL CHEMISTRY., vol. 4, no. 6, 28 September 2002 (2002-09-28), US, pages 549 - 551, XP093090256, ISSN: 1520-4766, DOI: 10.1021/cc020044z

## Description

### Field of the Invention

The present invention relates to cosmetic compositions for maintaining and restoring skin barrier function, especially in aging skin.

### Background of the Invention

As the outermost layer of the epidermis, the stratum corneum is the first line of defence for the body, serving an essential role as a protective skin barrier against the external environment.

A diverse set of strategies is used by the stratum corneum to maintain epidermal integrity. The complex tissue of the stratum corneum supports execution of these strategies and is composed of corneocytes and a matrix of intercellular lipids (ceramide, cholesterol, and free fatty acids), with both components derived from the terminal differentiation process of keratinocytes. Evidently, proper development and maintenance of the stratum corneum are essential for maintaining its barrier function.

Like every other complex biological system, the skin grows, matures, and ages throughout the life course. With increasing age, the skin undergoes changes in appearance and function, referred to as intrinsic or chronologic aging, and these are exaggerated by exposure to external factors such as UVR. Studies show that the epidermal barrier of the skin is perturbed during aging, and following insult there is decreased recovery of function. Several mechanisms may underlie this functional decline, including loss of specific homotypic cell adhesion proteins, as well as alterations in cytokine signaling and lipid content.

It is generally accepted that an impairment of skin barrier function is a central event in various problem skin conditions including dryness and sensitivity, as well as inflammatory skin disorders such as atopic dermatitis and eczema. Epidermal dysfunction is also thought to contribute to the development of chronic, low-grade systemic inflammation, termed 'inflammaging,' which is linked to the development of aging-associated systemic disorders.

US 2021/145723 A1 discloses the use of N(4-amino-2-methylquinolin-6-yl)-2-((4-ethylphenoxyl)methyl)benzamide against skin aging, which is explained is also connected with the barrier function of the skin.

Thus, there exists a need for compositions for maintaining and restoring skin barrier function, especially in aging skin.

The present invention addresses this problem.

### Summary of the Invention

The invention provides a cosmetic composition for maintaining and restoring skin barrier function comprising one or more benzamide compounds of general formula (I): 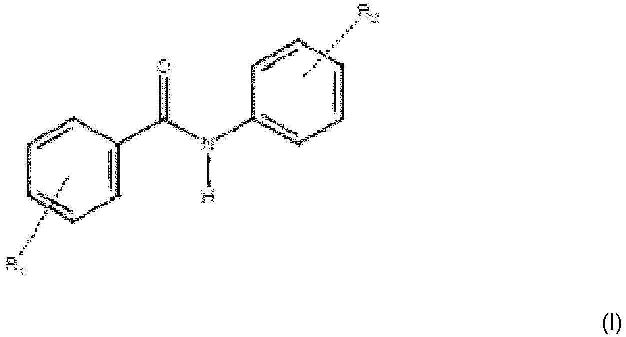 in which R₁ is a C₁₋₄ alkyl or a benzamido group, and R₂ is a C₁₋₄ alkoxy group.

The invention also provides a cosmetic method for maintaining and restoring skin barrier function comprising applying the cosmetic composition, as defined above, to an area of skin to be treated.

The invention also provides the cosmetic use of one or more benzamide compounds of general formula (I), as defined above, as active ingredients for maintaining and restoring skin barrier function.

### Detailed Description of the Invention

### Definitions

As used herein, the term "skin" denotes the skin of the face, of the body and of the scalp.

As used herein, the term "skin barrier function" denotes the function of the skin to protect a subject from external hazards, including microbial, chemical and/or mechanical insults. A variety of invasive and noninvasive *in vitro* and *in vivo* methods for measuring skin barrier function are described in the literature. the most widely used being the measurement of the amount of transepidermal water loss (TEWL).

As used herein, "skin care" means regulating and/or improving cosmetic qualities of the skin, as opposed to curing, treating, or preventing a disease or disorder. Accordingly, such cosmetic qualities are subject to regulation and/or improvement both in healthy subjects as well as those which present diseases or disorders of the skin (such as psoriasis, lichen planus, folliculitis, or atopic dermatitis).

### Benzamide compounds of general formula (I)

In general formula (I) as defined above, R₁ is preferably a methyl group or a benzamido group. In general formula (I) as defined above, R₂ is preferably a methoxy group.

Specific examples of preferred benzamide compounds of general formula (I) include 3-benzamido-N-(4-methoxyphenyl) benzamide and N-(3-methoxyphenyl)-4-methylbenzamide. 3-benzamido-N-(4-methoxyphenyl) benzamide is particularly preferred.

Mixtures of any of the above-described materials may also be used.

### Product Forms

The cosmetic composition according to the invention can be formulated in a variety of forms for topical application and will generally contain from about 0.01 to about 10%, preferably from about 0.1 to about 5% of the one or more benzamide compounds of general formula (I) (by weight based on the total weight of the composition).

Topical cosmetic compositions according to the invention will generally include a cosmetically acceptable vehicle. The term "cosmetically acceptable" means that the vehicle is suitable for topical application to the skin, has good aesthetic properties, is compatible with the one or more benzamide compounds of general formula (I) and any other ingredients, and will not cause any safety or toxicity concerns.

The vehicle may comprise an aqueous phase, an oil phase, an alcohol, a silicone phase, or a mixture thereof, and may be in the form of an emulsion. Emulsions can have a range of consistencies including thin lotions (which may also be suitable for spray or aerosol delivery), creamy lotions, light creams, and heavy creams.

Exemplary emulsions include water-in-oil emulsions, oil-in-water emulsions, silicone-in-water emulsions, water-in-silicone emulsions, polyol-in-silicone emulsions, silicone-in-polyol emulsions, polyol-in-oil emulsions, oil-in-polyol emulsions, wax-in-water emulsions, and water-oil-water triple emulsions. Preferred emulsions include oil-in-water emulsions and water-in-oil emulsions.

Topical cosmetic compositions according to the invention and in the form of an emulsion typically have an oil phase containing at one or more cosmetically acceptable fatty materials which may be liquid or solid at room temperature (25°C).

Suitable cosmetically acceptable fatty materials include naturally derived oils (such as sunflower oil, borage oil, soybean oil, castor oil, olive oil and almond oil); esters of monoalcohols or of glycols with monocarboxylic or polycarboxylic acids, at least one of the alcohols and/or acids comprising at least one hydrocarbon-based chain containing at least 6 carbon atoms (such as octyl palmitate, isopropyl myristate, isopropyl palmitate, isopropyl isostearate, hexyl laurate, isohexyl laurate, isohexyl palmitate, decyl oleate, isodecyl oleate, hexadecyl stearate, decyl stearate, dihexyldecyl adipate, lauryl lactate, myristyl lactate, cetyl lactate, oleyl stearate, oleyl oleate, oleyl myristate, lauryl acetate, cetyl propionate, isononyl isononanoate, propylene glycol dicaprate, diisopropyl adipate, dibutyl adipate, and oleyl adipate); ethers (such as dicapryl ether); fatty alcohols (such as cetyl alcohol, stearyl alcohol and behenyl alcohol); isoparaffins (such as isooctane, isododecane and isohexadecane); silicone oils (such as dimethicones, cyclic silicones, and polysiloxanes); and hydrocarbon oils (such as mineral oil, petrolatum and polyisobutene); fatty acids containing from 8 to 30 carbon atoms, (such as stearic acid, lauric acid, palmitic acid and oleic acid); vegetable fats (such as cocoa butter, coconut oil, palm oil and shea butter) ; natural or synthetic waxes (such as lanolin wax, beeswax, carnauba wax, candelilla wax, paraffin wax, lignite wax, microcrystalline waxes, ceresin, ozokerite, and polyethylene waxes), hydrogenated oils which are solid at 25° C (such as hydrogenated castor oil, hydrogenated jojoba oil, hydrogenated palm oil, hydrogenated tallow and hydrogenated coconut oil) and fatty esters that are solid at 25°C (such as C₂₀₋₄₀ alkyl stearate).

Mixtures of any of the above-described materials may also be used.

Topical cosmetic compositions according to the invention and in the form of an emulsion typically have an aqueous phase, with the amount of water in such an emulsion suitably ranging from about 5 to about 95%, preferably from about 35 to about 80% (by weight based on the total weight of the composition). The aqueous phase may also include one or more organic liquids that are miscible with water at room temperature (25°C). Exemplary water-miscible organic liquids include monohydric and polyhydric alcohols and derivatives thereof such as C₂-C₆ alkanols (such as ethanol and isopropanol); C₂-C₁₀ glycols and polyols (such as glycerol, propylene glycol, butylene glycol, pentylene glycol, hexylene glycol, caprylyl glycol, dipropylene glycol, and diethylene glycol); C₃-C₁₆ glycol ethers (such as mono-, di-, or tripropylene glycol (C₁-C₄) alkyl ethers and mono-, di-, or triethylene glycol (C₁-C₄) alkyl ethers) and polyethylene glycol having 2 to 12 oxyethylene units.

Topical cosmetic compositions according to the invention and in the form of an emulsion generally include emulsifiers and solubilizers, to enable two or more immiscible components to be combined homogeneously and to help stabilize the composition. The amount of emulsifier and solubilizer in such an emulsion suitably ranges from about 0.1 to about 30%, preferably from about 1 to about 8% (by weight based on the total weight of the composition). Emulsifiers that may be used to form O/W or W/O emulsions include sorbitan oleate, sorbitan sesquioleate, sorbitan isostearate, sorbitan trioleate, PEG-20 sorbitan isostearate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, polyglyceryl-2 dipolyhydroxystearate, PEG-30 dipolyhydroxystearate, oleamide DEA, TEA myristate, TEA stearate, magnesium stearate, sodium stearate, potassium laurate, potassium ricinoleate, sodium cocoate, sodium tallowate, potassium castorate, sodium oleate, cetyl phosphate, diethanolamine cetyl phosphate, potassium cetyl phosphate, sodium glyceryl oleate phosphate, dimethicone copolyol, cetyl dimethicone copolyol, octyldimethicone ethoxyglucoside copolyol, dimethicone copolyol crosspolymer and laurylmethicone copolyol.

Mixtures of any of the above-described materials may also be used.

Topical cosmetic compositions according to the invention may also take the form of a skin cleanser incorporating one or more cleansing surfactants which, when combined with water and mechanically agitated, generate a foam or lather. The amount of cleansing surfactant suitably ranges from about 5 to about 40%, preferably from about 10 to about 35% (by weight based on the total weight of the composition). Exemplary cleansing surfactants include anionic surfactants such as ammonium lauroyl sarcosinate, sodium trideceth sulfate, sodium lauroyl sarcosinate, sodium myristoyl sarcosinate, ammonium laureth sulfate, sodium laureth sulfate, ammonium lauryl sulfate, sodium lauryl sulfate, ammonium cocoyl isethionate, sodium cocoyl isethionate, sodium lauroyl isethionate, sodium cocoyl glycinate, sodium lauroyl glycinate, sodium methyl cocoyl taurate, sodium methyl lauroyl taurate, sodium methyl oleoyl taurate, sodium cetyl sulfate, sodium lauroyl lactylate and triethanolamine lauroyl lactylate and mixtures thereof; nonionic surfactants such as lauramine oxide, cocoamine oxide, decyl polyglucose, lauryl polyglucose, sucrose cocoate and sucrose laurate and mixtures thereof; and amphoteric surfactants such as disodium lauroamphodiacetate, sodium lauroamphoacetate, cetyl dimethyl betaine, cocoamidopropyl betaine and cocoamidopropyl hydroxy sultaine and mixtures thereof.

Mixtures of any of the above-described materials may also be used.

Topical cosmetic compositions according to the invention may also be formulated in a single-phase carrier such as water and/or one or more water miscible organic liquids (such as the monohydric and polyhydric alcohols and derivatives thereof described above).

Topical cosmetic compositions according to the invention may also be formulated in solid forms such as gels or sticks.

Combinations of any of the above-described product forms may also be used.

### Skin Care Actives

A topical cosmetic composition according to the invention may include additional skin care actives.

Examples of skin care benefits in the context of this invention include providing a smoother, more even texture; improving the elasticity or resiliency of the skin; improving the firmness of the skin; improving the hydration status or moisturization of the skin and reducing the appearance of redness or skin blotches.

Examples of additional skin care actives which are suitable for use in the composition according to the invention include vitamins, minerals and/or antioxidants, emollients, humectants, skin anti-hyperpigmentation agents, sunscreens, anti-irritants, exfoliating agents, and mixtures thereof.

Vitamins, minerals and/or antioxidants suitable for use in the composition according to the invention include natural botanical antioxidants derived from plant materials such as fruits, vegetables, herbs and spices (such as goji berry, white tea, rosemary, green tea, grape seed and lemongrass extracts); vitamin A and its precursors or derivatives (such as beta-carotene, retinyl palmitate); vitamin B3 and its precursors or derivatives (such as niacinamide); vitamin B5 and its precursors or derivatives (such as panthenol and its precursors or derivatives); vitamin C and its precursors or derivatives (such as tetrahexyldecyl ascorbate, ascorbyl palmitate); vitamin E and its precursors or derivatives (such as d-alpha-tocopherol, tocopheryl acetate); vitamin K and its precursors or derivatives; selenium and its derivatives (such as L-selenomethionine); and alpha lipoic acid.

Emollients suitable for use in the composition according to the invention act to increase and maintain moisture in the skin by providing a protective coating to impede epidermal water loss. Examples of emollients include C₁₀₋₂₀ fatty alcohols and acids (such as cetyl, myristyl, palmitic and stearyl alcohols and acids); and C₁₀₋₄₀ hydrocarbons (such as mineral oil, petroleum jelly, squalene and isoparaffins).

Humectants suitable for use in the composition according to the invention act to increase and maintain moisture in the skin by attracting water to the stratum corneum of the epidermis. Examples of humectants include amino acids, chondroitin sulfate, glycerin, diglycerin, triglycerin, polyglycerin, polypropylene glycol, polyethylene glycol, ethylene glycol, diethylene glycol, triethylene glycol, propylene glycol, dipropylene glycol, hexylene glycol, 1,3-butylene glycol, 1,4-butylene glycol, ethylene glycol monoalkyl ether, diethylene glycol monoalkyl ether, erythritol, fructose, glucose, maltose, sucrose, lactose, xylose, inositol, lactitol, xylitol, sorbitol, mannitol, maltitol, panthenol, pentaerythritol, 1,2,6-hexanetriol, honey, hyaluronic acid, hydrogenated honey, hydrogenated starch hydrolysate, natural moisturization factor, PEG-15 butanediol, polyglyceryl sorbitol, sodium and potassium salts of pyrollidone carboxylic acid, sodium glucuronate, trehalose and urea.

Skin anti-hyperpigmentation agents suitable for use in the composition according to the invention include natural botanical agents derived from plant materials (such as *Arctostaphylos patula* and *Arctostaphylos viscida* extracts, *Emblica officinalis* extract, *Mitracarpus scaber* extract, *Uva ursi* (bearberry) extract, *Morus bombycis* (mulberry) extract, *Morus alba* (white mulberry) extract, *Broussonetia papyrifera* (paper mulberry) extract, licorice extract, acerola cherry extract, *Chlorella vulgaris* extract, *Aloe ferrox* extract and *Rumex occidentalis* extract); synthetic or natural sugar amines (such as glucosamine, N-acetyl glucosamine, glucosamine sulfate, mannosamine, N-acetyl mannosamine, galactosamine, N-acetyl galactosamine and their hydrochloride salts); retinoids (such as retinol, retinal, retinoic acid and C₂₋₂₀ esters of retinol such as retinyl palmitate, retinyl acetate, retinyl propionate, retinyl linoleate and retinyl oleate); kojic acid and C₂₋₂₀ esters thereof (such as kojic acid monobutyrate, kojic acid monocaprate, kojic acid monopalmitate, kojic acid monostearate and kojic acid monobenzoate); hydroquinone, hydroquinone monomethyl ether, hydroquinone monoethyl ether, hydroquinone monobenzyl ether, α and β-arbutin, deoxyarbutin (4-[(tetrahydro-2H-pyran-2-yl)oxy]phenol), mequinol (4-hydroxyanisole), glutathione, cysteine, N-acetyl-L-cysteine, azelaic acid, magnesium ascorbyl phosphate, sodium ascorbyl phosphate, peroxides (such as hydrogen peroxide, zinc peroxide, sodium peroxide and benzoyl peroxide); 3-aminotyrosine, glycyrrhizinic acid and 4-substituted resorcinol derivatives (such as 4-methyl resorcinol, 4-ethyl resorcinol, 4-propyl resorcinol, 4-isopropyl resorcinol, 4-butyl resorcinol, 4-pentyl resorcinol, 4-hexyl resorcinol, 4-heptyl resorcinol, 4-octyl resorcinol, 4-nonyl resorcinol, 4-decyl resorcinol, 4-undecyl resorcinol, 4-dodecyl resorcinol, 4-cyclopentyl resorcinol, 4-cyclohexyl resorcinol, 4-cycloheptyl resorcinol, and 4-cyclooctyl resorcinol).

Sunscreens suitable for use in the composition according to the invention protect the skin from ultraviolet (UV) solar radiation falling within both the UVB region (between 290nm to 320 nm wavelengths) and the UVA region (between 320nm and 400nm wavelengths). Examples of sunscreens include methoxycinnamate derivatives (such as octyl methoxycinnamate and isoamyl methoxycinnamate); camphor derivatives (such as 4-methyl benzylidene camphor, camphor benzalkonium methosulfate, and terephthalylidene dicamphor sulfonic acid); salicylate derivatives (such as octyl salicylate and homosalate); sulfonic acid derivatives (such as phenylbenzimidazole sulfonic acid); benzone derivatives (such as dioxybenzone, sulisobenzone, and oxybenzone); benzoic acid derivatives (such as aminobenzoic acid and octyldimethyl para-amino benzoic acid); octocrylene, diethylhexyl butamido triazone, octyl triazone, butyl methoxydibenzoyl methane, drometrizole trisiloxane, menthyl anthranilate and inorganic UV-absorbing particles (such as zinc oxide and titanium dioxide).

Anti-irritants suitable for use in the composition according to the invention include allantoin, aloe vera, α-bisabolol, caffeine, chamomile extract, *Cola nitada* extract, cucumber extract, dipotassium glycyrrhizinate, glycyrrhizic acid, green tea extract, lecithin or hydrogenated lecithin, licorice extract, *Avena sativa* (oat) meal extract, tea tree oil, salicylic acid, acetylsalicylic acid, strontium acetate, strontium chloride, strontium nitrate, fatty acids with anti-irritant properties (such as linoleic acid and linolenic acid) and aromatic aldehydes with anti-irritant properties (such as 4-methoxy benzaldehyde, 4-ethoxy benzaldehyde, 4-butoxy benzaldehyde and 4-pentoxy benzaldehyde).

Exfoliating agents suitable for use in the composition according to the invention include benzoyl peroxide, benzoic acid, 3-hydroxy benzoic acid, salicylic acid, acetic acid, trichloroacetic acid, 1-pyrrolidone-5-carboxylic acid, α-hydroxy acids (such as glycolic acid, lactic acid, malic acid, tartaric acid, and citric acid); β-hydroxy acids (such as β-hydroxybutyric acid); α-keto acids (such as pyruvic acid, 2-oxopropanoic acid, 2-oxobutanoic acid and 2-oxopentanoic acid); and oxa acids (such as 3,6,9-trioxaundecanedioic acid).

Mixtures of any of the above-described materials may also be used.

### Functional Ingredients

A topical cosmetic composition according to the invention may also include additional functional ingredients for improving the physical and/or aesthetic characteristics of the composition.

Examples of additional functional ingredients which are suitable for use in the topical cosmetic composition according to the invention include water soluble or colloidally water soluble polymeric thickening agents (such as hydroxyethyl cellulose, methyl cellulose, hydroxypropyl methyl cellulose, polyquaternium-10, carrageenan, guar gum, hydroxypropyl guar gum, xanthan gum, polyvinylalcohol, acrylic acid/ethyl acrylate copolymers, carboxyvinyl polymers, cross-linked polyacrylate polymers and polyacrylamide polymers); structurant clays (such as magnesium aluminum silicate, attapulgite, bentonite, montmorillonite and hectorite); inorganic pigments (such as titanium oxide, zirconium oxide, cerium oxide zinc oxide, iron oxide, chromium oxide and ferric blue); organic pigments (such as carbon black and barium, strontium, calcium, and aluminium lakes); pearlescent agents (such as mica coated with titanium oxide and/or iron oxide); dyes, preservatives (such as disodium EDTA, benzyl alcohol, methylparaben, phenoxyethanol, propylparaben, ethylparaben, butylparaben and isobutylparaben); pH adjusters and fragrances.

Mixtures of any of the above-described materials may also be used.

### Packaging

A topical cosmetic composition according to the invention may be packaged in a suitable container to suit its viscosity and intended use by the consumer. For example, a lotion or a cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar.

### Use

The topical cosmetic composition according to the invention may suitably be applied to the skin at the rate of one or two applications per day. Generally, an amount corresponding to about 1 to 2 ml of the composition per application is applied uniformly over the area of treatment twice daily for a period of at least 7 (seven) days, more preferably at least 30 (thirty) days.

The invention will be further illustrated by the following, non-limiting Examples.

### Examples

Barrier function and development are keys to proper skin function and health. A comparative series of gene assays were performed on barrier function related genes to show the performance of compounds of general formula (I) according to the present invention.

Primary keratinocytes from 5 donors were pooled and plated at 100,000 cells/well in 6 well plates. Cells were grown in KGM-Gold (Lonza) at 37°C, 5% CO₂ and then differentiated for 72hrs via the addition of 1.2mM Ca²⁺. Cells were then treated for 24hrs with fresh medium containing vehicle, or vehicle plus test compound, using a test compound concentration of 10µm. Following treatment any effect on cell viability was confirmed via an LDH assay on the culture media. The cells were washed in PBS and RNA isolated via QIAzol (QIAgen) following the manufacturer's instructions. RNA quantity and quality was measured via Nanodrop and bioanalyzer assessments ahead of qPCR assessment for the levels of specific gene products. Test genes were selected to cover various aspects of epidermal function and quantified relative to the levels of a control panel of housekeeper genes. A positive fold change (log2 FC) value indicates an increase of expression, while a negative fold change indicates a decrease in expression.

The genes assayed, and their expression profiles are shown in Table 1.

**Table 1**

| **Compound of general formula (I)** | **Test Gene** | | | | | | | | | **Average log2 FC** |
|---|---|---|---|---|---|---|---|---|---|---|
| | ***ALOX12B*** | ***CERS3*** | ***ELOVL4*** | ***CLDN1*** | ***FLG*** | ***KLK11*** | ***KRT10*** | ***LOR*** | ***TGM1*** | |
| | **log2 FC** | | | | | | | | | |
| 3-benzamido-N-(4-methoxyphenyl) benzamide | 0.277 | 0.424 | 0.301 | 0.416 | 0.482 | 0.342 | 0.476 | 0.567 | 0.497 | **0.420** |
| N-(3-methoxyphenyl)-4-methylbenzamide | 0.165 | 0.558 | 0.583 | 0.459 | 0.775 | 0.224 | 0.892 | 0.583 | 0.596 | **0.537** |

Molecules encoded by *ALOX12B, CERS3* and *ELOVL4* are involved in the synthesis, transport, and metabolism of skin specific ceramides which contribute to the formation of the corneocyte lipid envelope (CLE), and which are therefore critical for skin barrier function.

Molecules encoded by *KLK11, KRT10, LOR* and *TGM1* are involved in processes leading to the maturation of the stratum corneum and are therefore also critical for skin barrier function.

FLG encodes filaggrin, a protein that is central to the integrity of the skin barrier. Filaggrin contributes to the protein-lipid cornified cell envelope (CCE) which replaces the plasma membrane of differentiating keratinocytes and is extensively cross-linked by transglutaminases. Filaggrin is ultimately deiminated and degraded on the skin surface to release its component amino acids. A mixture of hygroscopic amino acids is produced, the so-called natural moisturising factor (NMF) which contributes to epidermal hydration and skin barrier function.

*CLDN1* encodes claudin-1, a protein which forms the tight junctions between the stratum granulosum and the stratum corneum and which is therefore essential for the integrity of the skin barrier.

The results in Table 1 above demonstrate that the compounds of general formula (I) according to the present invention provide a marked increase of expression in all the barrier function related genes which were assayed.

The following Table 2 illustrates cosmetic compositions for maintaining and restoring skin barrier function according to the present invention.

**Table 2**

| Skin Care Lotion | | |
|---|---|---|
| **Ingredient** | **Example 1** | **Example 2** |
| | **wt.% (a.i.)** | |
| Alkyl acrylate crosspolymer | 0.2 | 0.2 |
| Xanthan gum | 0.05 | 0.05 |
| Glycerin | 10 | 10 |
| Magnesium aluminum silicate | 0.3 | 0.3 |
| 3-benzamido-N-(4-methoxyphenyl)benzamide | 0.5 | - |
| N-(3-methoxyphenyl)-4-methylbenzamide | - | 0.5 |
| Stearic acid | 3.6 | 3.6 |
| Glycol stearate/stearamide AMP | 0.9 | 0.9 |
| Glyceryl monostearate | 0.8 | 0.8 |
| Cetyl alcohol | 0.5 | 0.5 |
| PEG stearate | 0.7 | 0.7 |
| Isopropyl myristate | 1.5 | 1.5 |
| Mineral oil | 1 | 1 |
| Dimethicone | 0.8 | 0.8 |
| Petrolatum | 0.5 | 0.5 |
| Tapioca starch | 0.5 | 0.5 |
| Hydroxyethyl urea | 0.02 | 0.02 |
| Water, fragrance, preservative, pH adjuster, chelating agent | to 100 | |

## Claims

1. The cosmetic, non-therapeutic use of one or more benzamide compounds of general formula (I): 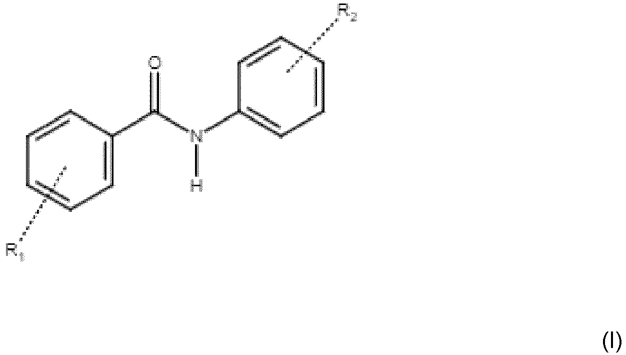 in which R₁ is a C₁₋₄ alkyl or a benzamido group, and R₂ is a C₁₋₄ alkoxy group, as active ingredients for maintaining and restoring skin barrier function.

2. Use according to claim 1 in which the benzamide compounds of general formula (I) are selected from 3-benzamido-N-(4-methoxyphenyl) benzamide and N-(3-methoxyphenyl)-4-methylbenzamide and mixtures thereof.

3. Use according to claim 2 in which the benzamide compound of general formula (I) is 3-benzamido-N-(4-methoxyphenyl) benzamide.

## Patentansprüche

1. Kosmetische, nichttherapeutische Verwendung einer oder mehrerer Benzamidverbindungen der allgemeinen Formel (I): 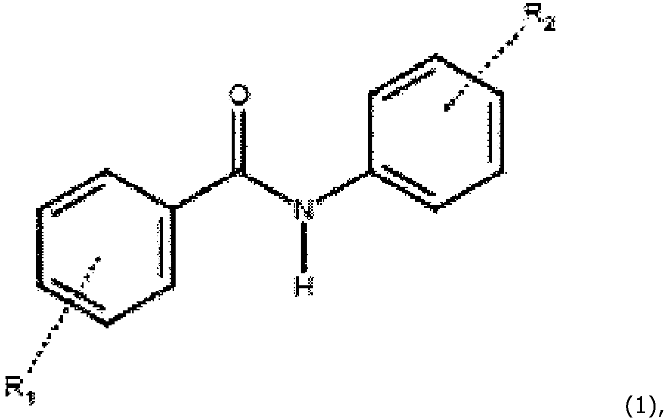 worin R₁ eine C₁₋₄-Alkyl- oder eine Benzamidogruppe ist und R₂ eine C₁₋₄-Alkoxygruppe ist, als Wirkstoffe zur Aufrechterhaltung und Wiederherstellung der Hautbarrierefunktion.

2. Verwendung nach Anspruch 1, wobei die Benzamidverbindungen der allgemeinen Formel (I) unter 3-Benzamido-N-(4-methoxyphenyl)benzamid und N-(3-Methoxyphenyl)-4-methylbenzamid sowie Mischungen davon ausgewählt sind.

3. Verwendung nach Anspruch 2, wobei die Benzamidverbindung der allgemeinen Formel (I) 3-Benzamido-N-(4-methoxyphenyl)benzamid ist.

## Revendications

1. Utilisation cosmétique, non thérapeutique d'un ou plusieurs composés de benzamide de formule générale (I): 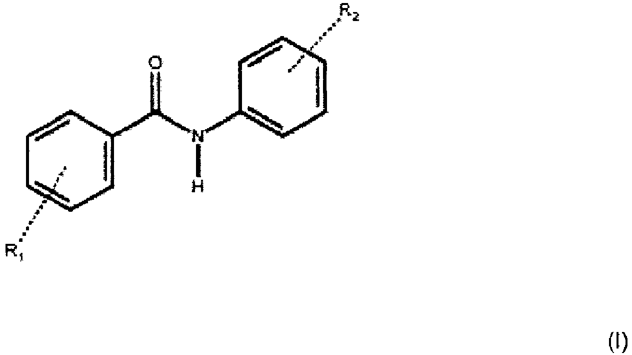 dans laquelle R₁ est un alkyle en C₁₋₄ ou un groupe benzamido, et R₂ est un groupe alcoxy en C₁₋₄, en tant qu'ingrédients actifs pour maintenir et restaurer la fonction de barrière de la peau.

2. Utilisation selon la revendication 1 dans laquelle les composés de benzamide de formule générale (I) sont choisis parmi le 3-benzamido-N-(4-méthoxyphényl)benzamide et le N-(4-méthoxyphényl)-4-méthylbenzamide et leurs mélanges.

3. Utilisation selon la revendication 2 dans laquelle le composé de benzamide de formule générale (I) est le 3-benzamido-N-(4-méthoxyphényl)benzamide.
